(19) Europäisches Patentamt
European Patent Office
Office européen des brevets

(11) Publication number: 0 101 046 B1

(12) **EUROPEAN PATENT SPECIFICATION**

(45) Date of publication of patent specification: **06.08.86**

(51) Int. Cl.$^4$: **C 12 Q 1/44,** C 12 N 9/20

(21) Application number: **83107827.4**

(22) Date of filing: **09.08.83**

(54) **Methods, compositions and elements for the determination of lipase.**

(30) Priority: **11.08.82 US 407214**

(43) Date of publication of application:
**22.02.84 Bulletin 84/08**

(45) Publication of the grant of the patent:
**06.08.86 Bulletin 86/32**

(84) Designated Contracting States:
**BE CH DE FR GB IT LI SE**

(56) References cited:
**EP-A-0 019 253**
**GB-A- 858 176**
**US-A-3 748 265**
**US-A-3 986 930**
**US-A-3 992 158**

**CHEMICAL ABSTRACTS, vol. 93, no. 13, September 1980, page 514, column 1, no. 130594c, Columbus, Ohio, USA**

**CHEMICAL ABSTRACTS, vol. 79, no. 5, 6th August 1973, page 183, no. 28802t, Columbus, Ohio, USA, T.B. HIGERD et al.: "Isolation of two acetyl esterases from extracts of Bacillus subtilis"**

(73) Proprietor: **EASTMAN KODAK COMPANY**
**343 State Street**
**Rochester New York 14650 (US)**

(72) Inventor: **LiMuti, Charles Michael**
**1627 Walk-Lake Ont. Road**
**Hilton New York 14468 (US)**
Inventor: **Mauck, John Charles**
**184 Croydon Road**
**Rochester New York 14610 (US)**
Inventor: **Babb, Bruce Edward**
**450 French Road**
**Rochester New York 14618 (US)**

(74) Representative: **Brandes, Jürgen, Dr.rer.nat. et al**
**Thierschstrasse 8**
**D-8000 München 22 (DE)**

(56) References cited:
**MICROBIOLOGY ABSTRACTS, vol. 11, no. 2, February 1976, column 7, abstract no. 11A539, London, G.B., B.J. ABBOTT et al.: "Physical properties and kinetic behavior of a cephalosporin acetylesterase produced by Bacillus subtilis"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
**CHEMICAL ABSTRACTS, vol. 73, no. 4, 27th July 1970, page 107, column 2, no. 16626b, Columbus, Ohio, USA, M. HATO et al.: "Solubility and critical micelle concentrations of fluorinated surfactants in water"**

**Description**

The present invention relates to methods, compositions and elements for the determination of lipase. The invention is particularly useful for the measurement of lipase in samples which also contain endogenous glycerol, such as blood serum and other body fluids.

Lipases which catalyze the hydrolysis of glycerol esters have been designated as glycerol ester hydrolases by the International Union of Biochemistry on the Nomenclature and Classification of Enzymes and have been given the designation Number E.C. 3.1.1.3.

The lipases determined according to the present invention are to be differentiated from other similar enzymes. These other similar enzymes include carboxylic ester hydrolases and aryl ester hydrolases. The carboxylic ester hydrolases catalyze the hydrolysis of the glycerol esters of short-chain fatty acids, as well as esters of monohydric alcohols and esters of dibasic acids. The aryl ester hydrolases catalyze the hydrolysis of esters such as phenyl acetate. Lipases, on the other hand, are specific for the glycerol esters of long-chain fatty acids and preferentially catalyze the hydrolysis of the ester on the end of the glycerol molecule. Lipases function only at an oil-water interface on these oily, water insoluble glycerol esters. The glycerol molecule has three possible ester positions with the two end positions being designated the α positions and the center position being designated the β position.

The determination of lipase in serum or other body fluids is an important aid in the diagnosis of various diseases. For example, elevated lipase in serum is observed in acute pancreatitis, duodenal ulcers and intestinal obstruction. In addition, elevated levels of lipase are found in some cases of carcinoma of the pancreas. Thus, it is readily apparent that the ability to determine the level of lipase in various fluids is an important diagnostic tool. In spite of the fact that the determination of lipase is valuable in the diagnosis of may diseases, assays for this enzyme are not frequently run in the clinical laboratory. The probable reason for this is that the known methods for the determination of lipase are relatively time-consuming and in may cases are inaccurate and nonspecific. For example, one of the most common methods is the turbidimetric method. To perform this method, an insoluble ester is emulsified. The sample to be analyzed is added to this turbid emulsion and the clearing effect that is caused by the lipase in the sample is measured. The most common insoluble ester used in this method is glycerol trioleate, also known as triolein. It is used either purified or directly as it is found in olive oil. It is known, however, that this method is relatively insensitive to small amounts of lipase in the sample an does not show good linearity at high enzyme levels. Further, it is not adaptable to dry analytical elements.

In another method for lipase determination, the fatty acids liberated as a result of the lipase-catalyzed hydrolysis of a fatty-acid ester are measured. A fresh batch of emulsion must be prepared for each lipase determination. The fatty-acid esters which are liberated are generally determined by titration, a labor-intensive procedure. While this method is accurate, it is extremely time-consuming and expensive. This method is also not adaptable to dry analytical elements.

Several colorimetric approaches have been suggested. For example, it has been suggested that certain phenol esters be used as the substrate for the lipase. In theory, as a result of the catalytic action of lipase, the naphthyl ester is hydrolyzed to a naphthol. Then, the liberated naphthol is reacted with another compound to produce a measurable dye. These processes involve several disadvantages. For example, the phenol esters are not specific substrates for lipase and it is likely that the aryl ester hydrolases which are also present in serum will catalyse the hydrolysis of these compounds. Thus, methods using such substrates are not specific for lipases and are therefore not clinically useful. One method of this type is described in U.S. Patent 3,616,251.

In another colorimetric method, an S-acyl compound is used as the lipase substrate. The compound reacts in the presence of lipase to produce an SH-group which then reacts with a chromogenic reagent to produce a color. Unfortunately, these substrates also react in the presence of carboxylesterases and acylesterases. This method is described in U.S. Patent 3,986,930.

The problem the present invention intends to solve is to provide a method for the determination of lipase which is quick and simple, avoids the many drawbacks of the previously known assays, such as the need to prepare fresh emulsions required by the turbidimetric and titrimetric methods and the lack of specificity of the known colorimetric methods and is adaptable to dry analytical elements.

The above problem is solved with a method for the determination of lipase in a sample, said method comprising the steps of:

(a) contacting said sample with a reagent composition and

(b) determining the rate of lipase characterized in that the reagent composition comprises

i) a lipase substrate which is a glycerol triester oil having in one of its two α-ester positions a long chain alkyl group having at least 8 carbon atoms and, in its two remaining ester positions, short chain alkyl groups such that, if the long chain alkyl group is hydrolyzed, the resulting diester is water soluble; and

ii) an esterase enzyme capable of catalyzing the hydrolysis of a water soluble glycerol diester to glycerol; and the lipase is determined by detecting the rate at which glycerol is formed.

The invention also provides a reagent composition for the determination of lipase comprising a lipase substrate characterized in that the composition comprises

(a) a lipase substrate which is a glycerol triester oil having in one of its two α-ester positions a long chain alkyl group having at least 8 carbon atoms and, in its remaining ester positions, short chain alkyl

groups such that, if the long chain alkyl group is hydrolyzed. The resulting diester is water soluble and

(b) an esterase enzyme capable of catalyzing the hydrolysis of a water soluble glycerol diester to glycerol.

The invention further provides a dry analytical element comprising a support and reagent zones for the determination of lipase, characterized in that the support has thereon:

(a) a zone comprising a lipase substrate which is a glycerol triester oil having in one of its two α-ester positions a long chain alkyl group having at least 8 carbon atoms and, in its remaining ester positions, short chain alkyl groups such that, if the long chain alkyl group is hydrolyzed, the resulting diester is water soluble and

(b) a zone comprising an esterase enzyme capable of catalyzing the hydrolysis of a water soluble glycerol diester to glycerol, said element comprising glycerol-detecting and -consuming reagents capable of producing a color change in the presence of glycerol.

The colorimetric assay provided by the invention is specific for lipase, easily adaptable to dry elements and uses stable components. The solution to the above problem involves a new series of reactions. The method of this invention comprises the determination of lipase in a sample, said method comprising the steps of:

(a) contacting said sample with a reagent composition and

(b) determining the rate of lipase characterized in that the reagent composition comprises

i) a lipase substrate which is a glycerol triester oil having in one of its two α-ester positions a long chain alkyl group having at least 8 carbon atoms and, in its two remaining ester positions, short chain alkyl groups such that, if the long chain alkyl group is hydrolyzed, the resulting diester is water soluble; and

ii) an esterase enzyme capable of catalyzing the hydrolysis of a water soluble glycerol diester to glycerol; and the lipase is determined by detecting the rate at which glycerol is formed.

The present invention is illustrated by reference to the sequence of reactions which takes place during the determination of lipase. The sequence of reactions, according to the process of the present invention, is represented schematically as follows:

a)

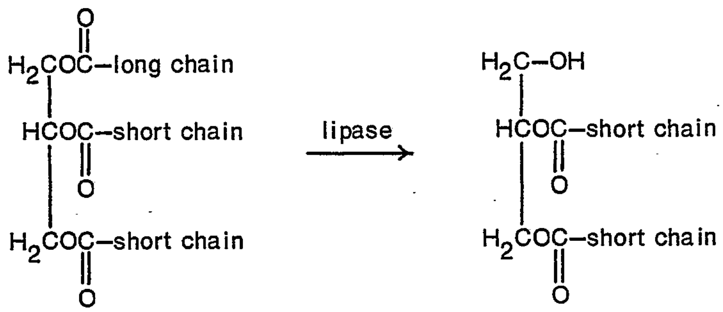


b)

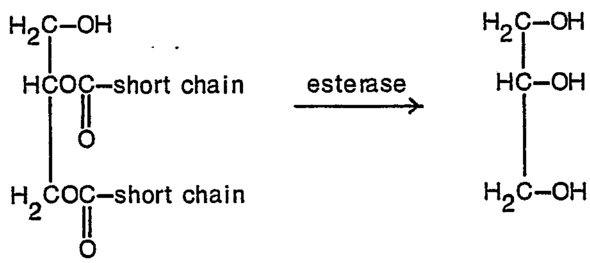


and

(c) measure rate of glycerol formation.

Referring to the above sequence, the specific lipase substrate which is used in the first step and the esterase which is used in the second step are critical to the invention. The esterase substrate is a glycerol triester oil having in one of its two α-ester positions a long chain alkyl group having at least 8 carbon atoms and, in its remaining ester positions, short chain alkyl groups such that, if the long chain alkyl group is hydrolyzed, the resulting diester is water soluble. The esterase used in the second step is an esterase enzyme capable of catlyzing the hydrolysis of a water-soluble glycerol diester to glycerol. Esterase enzymes useful in the present invention are active on water soluble glycerol diesters but are not active on oily glycerol esters. Therefore, compositions containing these two components are capable of being stored for long periods without concern that the esterase will act on the oily lipase substrate thereby reducing the sensitivity of the composition to lipase.

In particularly preferred embodiments, lipase is detected in a sample which contains endogenous glycerol. The reagent composition comprises, in addition to the specific substrate and esterase enzyme described above, glycerol-detecting and -consuming reagents which are capable of producing a color change in the presence of glycerol. According to these preferred embodiments, the final step in the process

is the detection of the rate of color change produced by the glycerol-detecting and -consuming reagents at a time after the glycerol-detecting and -consuming reagents have consumed substantially all of the endogenous glycerol which is present in the sample. It is particularly un-expected that a reaction sequence which relies on the detection of glycerol for quantitating lipase would be capable of doing so in the presence of endogenous glycerol.

In particularly preferred embodiments the reagent composition comprises glycerol-detecting and -consuming reagents.

In a preferred embodiment of the invention, the element comprises a support having thereon:

(a) a zone in the form of a layer comprising the described substrate for lipase,

(b) a zone in the form of a layer comprising the described esterase enzyme and

(c) glycerol-detecting and -consuming reagents. Preferably the lipase substrate and the glycerol-detecting and -consuming reagents are in separate zones.

While the composition and method of the present invention is described with particular reference to blood serum, it will be appreciated that the invention is also useful with other fluids such as urine and spinal fluid.

The reagent composition comprises a specific substrate for lipase. The substrate is a glycerol triester. In only one of the α positions of the glycerol triester is a long-chain alkyl group. This long-chain alkyl group has at least 8 carbon atoms, preferably from 8—20 carbon atoms. By providing a long chain in one of the α positions of the glycerol triester, the substrate is an oil and is therefore a specific substrate for lipase. Other enzymes which are present in a fluid to be tested do not catalyze a reaction involving this specific substrate. The remaining positions on the glycerol triester are short-chain alkyl groups such that, it the long chain alkyl group is hydrolyzed, the resulting diester is water soluble. By soluble is meant that the diester is soluble to the extent of being able to form a 0.1N clear solution. Usually, the short-chain groups have from 1 to 4 carbon atoms. After the lipase has catalyzed the hydrolysis of the long-chain group, the short chain groups are hydrolyzed to liberate acyl groups which result in glycerol through the aid of an esterase enzyme. Useful substrates within this definition are easily made by methods which are known in the art.

In particular preferred embodiments, the long chain group in the α-position of the glycerol triester has 8 to 20 carbon atoms and the short chain group has 1 to 2 carbon atoms. These substrates are preferred because the resulting diacetyl glycerol is more rapidly hydrolyzed to glycerol by the esterase enzyme in the second reaction. The currently preferred substrate is 1-oleyl-2,3-diacetoiyl glycerol also referred to herein as oleyl diacetin or simply ODA. Other preferred substrates include 1-myristoyl-2,3-diacetoyl glycerol and 1-octanoyl-2,3-diacetoyl glycerol.

The substrate composition which is used need not be a single purified compound but optionally is a mixture of compounds. One commercially available composition is Myvacet®, available from the Eastman Kodak Company. This composition is used as purchased or it is used after a purifying treatment such as by column chromotography.

The second component of the reagent composition is an esterase enzyme. The useful esterases are enzymes which are capable of catalyzing the hydrolysis of the two short-chain alkyl groups from the water soluble diester which results from the hydrolysis of the long-chain group. The hydrolyzed diester has substantially no lipase activity. By using this specific substrate and this specific enzyme, no glycerol is produced unless lipase is present in the sample. Any esterase as described is useful in the reagent composition. Useful esterases are sometimes referred to as "short chain" esterases and are found in wheat germ and orange peel and various microbial sources such as *Bacillus subtilis* (see T. B. Higerd and J. Spizizen, *J. Bact., 114*, pp.l 1184—1192, 1973), *Nocardia* (see E. F. Eubanks et al, *J. Bact., 120*, pp. 1133—1143, 1974), *Sclerotina fungus* (see Oi and Satomura, *Agr. Biol. Chem., 31*, pp. 561—568, 1967) and *Saccharomyces cerevesiae* (see Wheeler and Rose, *J. General Microbiology, 74*, pp. 189—192, 1973).

In a particularly preferred embodiment, the esterase is a diacetinase from the microorganism *Bacillus subtilis*. (This esterase is referred to as a "Diacetinase" since it rapidly catalyzes the hydrolysis of acetyl groups. While the enzyme is also capable of catalyzing the hydrolysis of other short chain alkyl groups, the rate is significantly slower.) One particularly preferred enzyme is BS diacetinase ATCC No. 31954. This enzyme is preferred because it is highly specific, highly active and substantially free of lipase activity.

In preferred embodiments, the reagent composition also contains, in addition to the specific substrate and specific enzyme described above, glycerol-detecting and -consuming reagents. These reagents are capable of producing a color change in the presence of glycerol and thus detect the presence of glycerol. In addition, these reagents produce the color change by consuming the glycerol. These compositions are useful for the assay of samples containing endogenous glycerol. The glycerol reagents consume substantially all of the endogenous glycerol and thereafter the rate of color change is measured and is related to the amount of lipase present.

Many such glycerol-detecting and -consuming reagents are known in the art. For example, glycerol is converted in the presence of adenosine triphospate (ATP) and glycerol kinase to produce α-glycerol phosphate and adenosine phosphate (ADP). The ADP, through a series of reactions, produces a color change through the conversion of reduced nicotinamide dinucleotide (NADH) to nicotinamide dinucleotide (NAD). This particular reaction and other similar reactions are disclosed in U.S. Patent 3,703,591. Other glycerol-detecting and -consuming reagents include glycerol oxidase. Reagents of this type are disclosed in U.S. Patent 4,255,519.

In particularly preferred embodiments, the glycerol-detecting and -consuming reagents include α-glycerophosphate oxidase (α-GPO). Reagents of this type are described in U.S. Patent 4,241,178. The glycerol-detecting and -consuming reagents include adenosine triphosphate (ATP), glycerol kinase, an electron acceptor such as oxygen, and α-GPO. These reagents produce hydrogen peroxide in the presence of glycerol, and the reagents optionally contain a reduced dye and peroxidase. Hydrogen peroxide in the presence of the reduced dye and peroxidase produces a detectable dye. According to the reaction sequence, glycerol and ATP react in the presence of glycerol kinase to produce α-glycerophyosphate. The α-glycerophosphate and the electron acceptor react in the presence of the α-GPO to produce dihydroxyacetone phosphate and a detectable species. Where oxygen is the electron acceptor, the detectable species is hydrogen peroxide. Specific components and their sources which are useful in the preferred glycerol-detecting and -consuming reagents are known in the art.

The preferred elements of this invention comprise coenzymes such as colipase. The use of colipase improves the accuracy of the assay and aids in decreasing interference from other sources.

The process and reagent composition according to the present invention is adaptable to both solution and dry element assays. Thus, a solution containing the described reagent composition is prepared and lipase is determined in a sample by simply adding the sample to the reagent composition. In a solution assay, one method of calculating the rate of glycerol formation is to sample the reaction mixture at several times during the reaction. Each sample is treated to stop the reaction. The rate of glycerol formation is then calculated from the amount of glycerol in each sample. The amount of glycerol is determined by any known method such as chromatographic methods. Alternatively, the reagent composition is included in a dry element such as is described in U.S. Patent 2,992,158 and lipase is determined in a sample by simply spotting the sample on the dry element. The rate of formation of color in a reagent layer containing the glycerol-detecting reagents is then related to the rate of glycerol formation which in turn is related to the lipase activity of the sample.

The substrate and enzyme, as well as other reagents, are incorporated in zones in the element. Useful elements include, for example, dip-and-read elements generally containing the reagents in discrete zones in a single layer, and elements wherein the zones are superimposed layers.

In preferred embodiments, the method of the present invention includes the step of measuring the rate of glycerol formation after the glycerol-detecting and -consuming reagents have consumed substantially all of the endogenous glycerol in the sample. Usually, endogenous glycerol is rapidly consumed and any rate of glycerol formation about 2 to 6 minutes after the sample is contacted with the reagent composition is essentially due only to the presence of lipase. In samples containing extremely high levels of endogenous glycerol the detection limit of the glycerol-detection reagents is approached. It is desirable to dilute such samples.

The concentration of various components in the glycerol-detecting and -consuming reagent composition varies over a wide range depending on the solution under assay, i.e., blood serum, diluted or undiluted, or other complex aqueous solutions such as urine which may contain lipases. Details regarding amounts of the glycerol-detecting and -consuming regents are found in references relating to these reagents, such as U.S. Patents 3,703,591, 4,255,519 and 4,241,178, referenced above.

Where dry elements are desired, the reagent composition components are present in the layers in a wide range of coverages. Generally, the esterase is present in an amount between 100—5000 $IU/m^2$, preferably 2000—5000 $IU/m^2$. The lipase specific substrate is generally present in an amount between 1—20 $g/m^2$, preferably 5—15 $g/m^2$.

The process of the invention is particularly suited to dry elements. Spotting of the sample of analyte on the element forms an emulsion of the oily lipase substrate *in situ*. Lipase activity in the sample converts the oily glycerol triester lipase substrate to a water soluble glycerol diester. The glycerol diester then diffuses out of the emulsion-containing layer into layers containing the esterase and glycerol-detecting and -consuming reagents. While the layer placement of the esterase is not critical, it is highly desirable that the glycerol-detecting and -consuming reagents be in a layer separate from the lipase substrate layer so that the color which is formed is easily detectable. In some cases it is desirable to separate the substrate-containing layer from the glycerol-detection layer with a spacer layer. A spacer layer of a hydrophilic colloid such as gelatin is useful for this purpose.

A variety of dry element formats are useful. In one embodiment, for example, a support is coated sequentially with a layer containing the glycerol-detecting and -consuming reagents, a layer containing the esterase, a layer containing the lipase substrate and a spreading layer. In an alternative embodiment, the support is coated with a layer which contains both the esterase enzyme and the glycerol-detecting and -consuming reagents, a gelatin spacer layer and a spreading layer which contains the lipase substrate.

The support for the dry elements is any dimensionally stably support, preferably transparent. Useful supports are described in U.S. Patent 3,992,158, and the preferred support is poly(ethylene terephthalate).

The layer containing the glycerol-detecting and -consuming reagents preferably includes the reagents described in U.S. Patent 4,241,178 referenced above. This reagent composition preferably contains peroxidase to catalyze the reaction of a leuco dye with hydrogen peroxide to form the colored dye. In addition to the materials described in U.S. Patent 4,241,178, this layer optionally contains a latex to stabilize the peroxidase, such as is described in U.S. Patent 4,283,491.

In preferred embodiments, the layer containing the esterase includes a hydrophilic binder, a buffer and

a surfactant. Similarly, in preferred embodiments the layer containing the lipase specific substrate includes binder, a surfactant and a coenzyme. Preferred surfactants for use in the substrate-containing layer are surfactant FC—143® which is a perfluorooctanoate ammonium salt available from the 3N Company and Siponate DS—10 (sodium dodecyl benzene sulfonate) from Alcolac Chemical Corp.

Other useful suractants for the substrate-containing layer include any surfactants which do not inhibit lipase activity. Useful surfactants include Triton X—200® the sodium salt of an alkyl aryl polyether sulfonate and sodium cholate. Other layers optionally contain surfactants such as those mentioned above and other surfactants such as octylphenyl polyethoxyethanols such as Triton X—100®. Triton® surfactants are available from the Rohm and Hass Company. The surfactant is present in an amount sufficient to improve the coatability of the layer and to improve the wetting of the layer by the spotted sample. It is usually between about 0.1—3.0 g/$m^2$.

Useful hydrophilic binders include any film-forming, sample-permeable material which does not affect the enzyme activity. Useful hydrophilic binders include those which are known in the photographic arts. Particularly useful binders include gelatin. Other useful binders include synthetic polymers such as polymers of acrylamide, such as poly(*n*-butylacrylate-co-*n*-isopropylmethacrylamide) sodium salt, poly(acrylamide), poly(acrylamide-co-*n*-vinyl-2-pyrrolidone) and polyvinyl pyrrolidone (hereinafter Binder A). The binder is present in an amount sufficient to suspend the reagents and form a continuous uniform layer. Usually, the binder is present in an amount between 2—15 g/$m^2$ in the reagent layer.

Useful buffers are those which are capable of maintaining a pH of between 7.5 and 9.5 at 37°C. Preferably, the buffer is capable of maintaining a pH of about 8.8, the pH optimum of most lipases. Useful buffers include tris(hydroxymethyl)aminomethane and its hydrochloride (hereinafter tris-HCl), and N,N-bis(2-hydroxyethyl)glycine (hereinafter bicine). The buffer is present in an amount sufficient to maintain the desired pH when the element is spotted with the sample to be analyzed. Usually, the buffer is present in an amount between 1.0 and 7.5 g/$m^2$.

The spreading layer which, in some embodiments, optionally contains the lipase substrate, is composed of any of a wide variety of materials. Particularly preferred spreading layers are disclosed in U.S. Patent 3,992,158 referenced above. Other useful spreading layers are described in U.S. Patent 4,258,001. Combinations of spreading layers are also useful. For example, a spreading layer with relatively large pores such as the spreading layer in U.S. Patent 4,258,001 is useful over a layer having relatively small pores, such as many of the layers disclosed in U.S. Patent 3,992,158, to separate and spread plasma from whole blood. In alternative embodiments, filter paper or cloth are laminated with the spreading layer of U.S. Patent 3,992,158, again to separate and spread plasma from whole blood.

One particularly suitable spreading layer includes a microcrystalline colloid material, such as microcrystalline cellulose and a small amount of a binder. This spreading layer has been found to be substantially inert with respect to human pancreatic lipase.

The described reagent compositions are optionally incorporated into a variety of elements which are well-known in the art. Useful materials and elements which are adapted to use the described reagent composition are described, for example, in U.S. Patents 3,092,465, 3,418,099, 3,418,083, 2,893,843, 2,893,844, 2,912,309, 3,008,879, 3,802,842, 3,798,064, 3,298,739, 3,915,647, 3,917,453, 3,933,594 and 3,936,357.

The following examples serve to illustrate certain embodiments of the present invention.

In the following examples, the assayed value for lipase is frequently compared with the value for lipase for the same sample from a reference procedure. The reference procedure is performed using a 0.25-ml burette filled with 10 millimolar sodium hydroxide. The temperature is maintained at 37°C. An amount of 5 ml of a triolein emulsion was added and the background hydrolysis rate (i.e., the rate in the absence of lipase) recorded. The sample suspected of containing lipase was added to this emulsion. The pH of the sample was maintained at 8.8 during the hydrolysis by continuous titration of the sample with sodium hydroxide. The rate of addition of sodium hydroxide is monitored on a chart recorder and is related to the lipase activity because the sodium hydroxide is added neutralize acid given off by the hydrolysis. This assay is described in "Proposed Standard Method for Measuring Lipase Activity in Serum by a Continuous Sampling Technique", Tietz et al,. *Clin. Chem.*, 19/11, 1268—1275 (1973).

Example 1:

A dry test element was prepared according to the following format:

| |
|---|
| Spreading-reagent layer:<br>Microcrystalline cellulose, Binder A, ODA |
| Gelatin Spacer:<br>gelatin, Triton® X-100, gelatin hardener |
| Glycerol detection-enzyme layer:<br>gelatin, Alkanol® XC, 2, 4-di-n-pentylpheno, dye precursor[1], latex[2], ATP, $MgCl{\cdot}6H_2O$, gelatin hardener, bicine buffer, KCl, peroxidase, glycerol kinase, α-GPO, BS diacetinase |
| Support:<br>poly(ethylene terephthalate) |

[1] [2(3, 5-dimethoxy-4-hydroxyphenyl)-4, 5-bis(4-di-methylaminophenyl) imidazole]
[2] poly(methacrylate-*co*-2-acrylamido-2-methylpropane sulfonic acid-*co*-2-acetoacetoxyethyl methacrylate)

The diacetinase in the above coating format is the enzyme which is prepared as follows:

A. Materials

Egg-white lysozyme, deoxyribonuclease (DN-100) from bovine pancreas, ribonuclease A from bovine pancreas (Type 1-A), triolein and gum arabic were purchased from Sigma Chemical Co., St. Louis, Mo. Bacto® yeast extract was obtained from Difco Labs, Detroit, Mich. DeMann, Ragossa and Sharpe (MRS) broth (CM 359), yeast extract, Lab Lemco® powder (L—29) (meat extract nutrient broth), peptone (L—34), and Oxoid agar III® were purchased from Oxoid Canada Ltd, Ottawa, Ontario, Canada. Polyglycol (P—2000)® was obtained from Dow Chemical Co., Midland, Mich. Diacetin, glucose and other chemicals, unless otherwise specified, were obtained from Eastman Organic Chemicals, Rochester, N.Y.

B. Media

MRS Medium

| | Per Liter |
|---|---|
| peptone (L—34) | 10.0 g |
| Lab Lemco® powder (L—29) | 8.0 g |
| yeast extract (Oxoid) | 4.0 g |
| glucose | 20.0 g |
| Tween® 80 surfactant | 1.0 ml |
| potassium hydrogen phosphate ($K_2HPO_4$) | 2.0 g |
| sodium acetate trihydrate | 5.0 g |
| triammonium citrate | 2.0 g |
| magnesium sulfate heptahydrate | 0.2 g |
| manganese sulfate tetrahydrate | 0.05 g |
| agar (Oxoid III®) | 20.0 g |

The pH was adjusted to 6.2 with dilute sulfuric acid.

Diacetin-Containing MRS Medium

| | Per Liter* |
|---|---|
| MRS medium described above | 72.25 g |
| diacetin (filter sterilized) | 2.0 ml |

Salt Solution C (Modified)

| | Per Liter |
|---|---|
| sodium chloride (NaCl) | 0.6 g |
| calcium chloride dihydrate | 0.1 g |
| ferric sulfate heptahydrate | 2.8 g |
| sodium molybdate dihydrate | 0.1 g |
| zinc sulfate heptahydrate | 0.06 g |
| manganese sulfate monohydrate | 1.7 g |
| Magnesium sulfate heptahydrate | 25.0 g |

* The starting solution was 0.1 N hydrochloric acid.

Yeast Extract Medium

| | Per Liter |
|---|---|
| ammonium sulfate | 2.0 g |
| potassium hydrogen phosphate ($K_2HPO_4$) | 2.0 g |
| yeast extract (Bacto) | 5.0 g |
| salt solution C (modified) | 10.0 ml |

The pH was adjusted to 6.9 with dilute sulfuric acid.

Diacetin-Containing Yeast Extract Medium

| | Per Liter |
|---|---|
| yeast extract medium (described above) | 9.0 g |
| diacetin (filter sterilized) | 2.0 ml |

Pyruvate Medium (PM)

| | Per Liter |
|---|---|
| sodium pyruvate | 10.0 g |
| yeast extract | 5.0 g |
| potassium hydrogen phosphate ($K_2HPO_4$) | 2.0 g |
| salt solution C (modified) (described above) | 10.0 ml |

The pH was adjusted to 4.5 with 6 N hydrochloric acid.

C. Procedures

1. Isolation of *Bacillus subtilis*

The culture was isolated from soil samples by enrichment in the pyruvate medium described above, at 40°C. Ten frozen soil samples were thawed. Approximately 10 g each of the soil samples were added to 125 ml flasks, containing 25 ml of pyruvate medium. The flasks were incubated at 40°C without shaking. When the media became turbid, in about 3 days, 0.5 ml of each was transferred to test tubes containing 10 ml of pyruvate medium. After several days of incubation at 40°C without shaking, each test-tube culture was diluted 10:1, plated out on pyruvate medium and MRS medium, which was supplemented with pyruvate at 0.25 per cent and then incubated for 24—48 hr. Of these cultures, isolate ATCC No. 31954 was chosen.

2. Maintenance and Growth of Cultures

Cultures were maintained on MRS medium. The MRS slants were incubated at 40°C in a rotary shaker-incubator and transferred weekly.

Growth of bacterial cultures was accomplished by placing 50 ml of the various culture media, described above, in 250 ml conical flasks, inoculating with cells from MRS slants above, and incubating in the Psycrotherm at 40°C and 200 rpm (2-inch throw) for 12 hr.

3. Disruption of Microbial Cells and Preparation of Cell-Free Extracts

Microbial cells were disrupted by lysozyme treatment according to the following procedure: A lysis reagent was prepared which contained 1 mg/ml lysozyme, 0.1 mg/ml deoxyribonuclease, and 0.1 mg/ml ribonuclease in 0.05 M potassium phosphate buffer at pH 7.0. A typical batch of cells from 6.6 l of medium had a wet weight of 50 g. This cell paste was suspended in 250 ml of lysis reagent (17 percent suspension) and brought to 37°C and incubated at that temperature for 30 min in a metabolic shaker rotating at 150 rpm. Cell-free extract was prepared by centrifugation at 39,000 xg for 10 min in a refrigerated centrifuge.

4. Production of the Enzyme

Seven liters of yeast-extract medium were prepared as described above. Six fractions of medium, 1 liter each, were placed in fernbach flasks and 1 drop of polyglycol antifoam added to each. Fifteen fractions of medium, 50.0 ml each, were placed in 250 ml conical flasks. All were autoclaved for 30 min. When the flasks cooled to 40°C, sterilized diacetin was added to the fernbach flasks (2.0 ml each) and to the conical flasks (5 drops each).

Each of two conical flasks above were inoculated with one loopful of *Bacillus subtilis* ATCC No. 31954 from an MRS slant (2—3 days old). The flasks were incubated in the shaker-incubator at 40°C and 200 rpm for 12 hr.

The flask with the best growth, i.e., most turbid, was used to inoculate 12 of the remaining flasks. Each of the 12 flasks was inoculated with a 2.0 ml of inoculum and incubated as described above. Each of the 6 fernbach flasks described above was then inoculated with the contents of 2 of the remaining flasks which were then incubated in the Psycrotherm at 40°C for 12 hr.

A fraction was obtained from each fernbach flask and 1:10 dilutions were made. The optical density of each was read at 660 nm. Cells were collected by centrifugation on a refrigerated centrifuge at 0—4°C and 9000 rpm for 15 min and then stored frozen.

5. Partial Purification of the Enzyme

Disruption of microbial cells was carried out by lysozyme treatment as described above. After lysis, the total (initial) volume of the suspension was measured. The suspension was stirred in an ice bath until the temperature of the suspension was below 10°C. Then, over a 15 min period, without removing cell debris, cold *n*-propanol (−20°C) was added to a concentration of 40 percent (V/V). The mixture was allowed to stir for 20 min after the last addition, then centrifuged at 10,000 rpm at 5°C for 10 min. The pellet (from the 40 percent propanol fraction) was removed and the clear yellow supernatant again placed in an ice bath and stirred. Again, over a 15 min period, cold *n*-propanol was added, bringing the *n*-propanol concentration to 60 percent (V/V). The mixture was allowed to stir for 30 min and then centrifuged as described above. The small yellow pellets (from the 40—60 percent propanol fraction) were collected, placed in a beaker, and suspended in cold (5°C) 0.05 M potassium phosphate buffer, at pH 7.0, by stirring for 45 min. The cloudy suspension was centrifuged at 39,000 xg at 5°C for 10 min to clarify. The clear, slightly yellow product was stored frozen.

6. Measurement of the Enzyme

Measurements of enzyme activity were made using pH-Stat® instrumentation (Radiometer, Copenhagen). Standard reaction mixtures contained, in a total volume of 5.0 ml, 5 µmoles calcium chloride (1mM) and substrates at various concentrations. The pH was adjusted to 7.5, and mixtures were equilibrated at 37°C. Reaction was initiated by enzyme addition and then pH maintained at 7.5 by additon of 10.4 mM sodium hydroxide. A blank rate was determined, and hydrolytic activity in each case was calculated from the net linear rate of addition of sodium hydroxide. One unit was that amount of enzyme which catalyzed the production of 1 µmole of acid (addition of 1 µmole NaOH) per min at 37°C and pH 7.5.

The following illustrates the growth of microorganism and production of esterase using various media:

A. Yeast-Extract Media

The *Bacillus subtilis* ATCC No. 31954 microorganism was grown in medium (a) yeast extract and medium (b) diacetin-containing yeast extract (both described above) according to the following procedure:

Fifty ml of medium (b) was inoculated with one loopful of *Bacillus* culture from an MRS slant and incubated in the shaker-incubator at 40°C and 200 rpm for 12 hr. Two ml of this 12 hr culture broth was used to inoculate a conical flask containing 50 ml of medium (a) and a conical flask containing 50 ml of medium (b). These flasks were incubated as described above.

Micorbial cells were isolated by centrifugation at 9000 rpm for 20 min at 0—4°C using a Sorvall® RC-2B refrigerated centrifuge.

B. MRS Media

Part A was repeated except that the culture was grown in medium (c) MRS and medium (d) diacetin-containing MRS.

Enzyme purification and assays were done as described above. The results tabulated in Table 1 indicate that medium (c) provided the best overall growth and enzyme production.

TABLE 1

| | Cell Growth (g wet wt/l) | Activities: Enzyme Production (U/l | Activities: Enzyme Production (U/g wet wt) |
|---|---|---|---|
| (a) yeast extract | 4.4 | 99 | 22.4 |
| (b) yeast extract + diacetin | 8.8 | 555 | 63.0 |
| (c) MRS | 8.4 | 657 | 78.0 |
| (d) MRS + diacetin | 5.6 | 371 | 68.0 |

Six levels of human pancreatic lipase calibrators were used to calibrate the dry test element. The calibrators were prepared by adding human pancreatic fluid to pooled human serum samples. The resulting calibrators were assayed using the reference method described above using trioleon as the substrate. Calibration of the element was performed by spotting the element with 10 µl samples of each

calibrator, incubating for 7 min at 37°C and reading the reflectance density at 670 nm using a reflectometer. The rate of color formation was determined for a 1-min time period at a point in a time after the endogenous glycerol in the sample had reacted with the glycerol-detecting and -consuming reagents. The 1-min period was between 5 and 6 min after spotting. The change in reflection density over the 1-min period ($\Delta D_R$/min) was plotted vs. the pancreatic lipase concentration (U/l) which was determined using the reference method. Using regression analysis, a linear response was determined which had a slope of $5.4 \times 10^{-5}$ $\Delta D_R$/min/U/l. The fact that a linear line with positive slope was determined indicates that it is possible to calibrate the method and therefore determine samples containing unknown amounts of lipase.

## Example 2

A dry test element was prepared according to the following format:

Spreading layer:
Microcrystalline cellulose Binder A

Reagent layer:
ODA[1], poly(acrylamide) binder, tris-HCl buffer, Triton® x-100

Enzyme Layer:
gelatin, wheat-germ esterase, tris-HCl buffer, Triton® x-100

Glycerol-detection-consuming layer:
gelatin, 2,4-di-*n*-pentylphenol, dye precursor (see Example 1)l latem (see Example 1), ATP $MgCl \cdot 6H_2O$, gelatin hardener, bicine buffer, KCl, peroxidase, glycerol kinase, α-GPO

Support:
poly(ethytlene)terephthalate

[1] The coating composition was an aqueous composition having 10% by volume ODA emulsified in 7% (wt/vol) gum arabic.

In a manner similar to Example 1, the element was calibrated using the human pancreatic lipase calibrators. A slope of $1.56 \times 10^{-5}$ $\Delta D_R$/min/U/l was calculated from the linearly regressed data points.

## Example 3
### Effects of Colipase in Element for the Determination of Lipase

Two dry test elements were prepared essentially the same except that one contained, in the spreading layer, 33,000 U/m² colipase (obtained from Boehringer Mannheim), and in the reagent layer, additional latex polymer.

The composition and format of the elements are as follows:

Spread Layer:
Titanium dioxide/cellulose acetate
Myvacet (substrate)
Siponate DS-10 (sodium dodecyl benzene sulfonate)
Colipase — 33,000 U/m² (in element I only)

Sub Layer:
Poly(n-isopropylacrylamide)

Gel Pad:
Gelatin
BVSME (hardener)
Triton X-200
*Latex Polymer
Ascorbate oxidase

Reagent Layer:
Diacetinase (esterase)
Gelatin
BVSME
Alkanol XC (surfactant)
*Latem polymer (in element I only)
α-Glycerophosphate oxidase
Peroxidase
Glycerol kinase
Bicine (buffer)
KCl
Dimedone
Adenosine triphosphate
$MgCl_2 \cdot 6H_2O$
2,4-di-n-pentylphenol (KS-52 solvent)
**Leuco dye

---

Support

---

* poly(methacrylate-co-2-acrylamido-2-methylpropane sulfonic acid-co-2-acetoacetoxy ethyl methacrylate)
** 2(3,5-dimethoxy-4-hydroxyphenyl)4,5-bis-(4-dimethylaminophenyl)imidazole

Calibrator fluids were comprised of hog pancreatic lipase in bovine serum albumin, 0—1790 U/L lipase. The reference assay was a modified pH stat method.

Patent samples comprised normal and abnormal values from 13-2700 U/L lipase.

Each element was spotted with 10μL samples, incubated for ~5 minutes, and reflectance densities were monitored at 540 nm. Calibration plots demonstrate the excellent correlation of values obtained between the element of the present invention and reference values. Also evident is the elimination of random bias from the element containing colipase.

Accuracy plots of these data are also determined. Results were 100% within accuracy goals when colipase was present, compared to 48.8% within accuracy goals in its absence.

Colipase improves the accuracy of the assay, and lowers bias from interferences such as endogenous glycerol by increasing the sensitivity of the assay composition to lipase.

**Claims**

1. A method for the determination of lipase in a sample, said method comprising the steps of:
(a) contacting said sample with a reagent composition and
(b) determining the rate of lipase characterized in that the reagent composition comprises
i) a lipase substrate which is a glyerol triester oil having in one of its two α-ester positions a long chain alkyl group having at least 8 carbon atoms and, in its two remaining ester positions, short chain alkyl groups such that, if the long chain alkyl group is hydrolyzed, the resulting diester is water soluble; and
ii) an esterase enzyme capable of catalyzing the hydrolysis of a water soluble glycerol diester to glycerol; and the lipase is determined by detecting the rate at which glycerol is formed.

2. The method of claim 1 characterised in that the reatent composition comprises:
i) a lipase substrate which is a glyerol triester oil having in one of its two α-ester positions a long chain alkyl group having at least 8 carbon atoms and, in its two remaining ester positions, short chain alkyl groups such that, if the long chain alkyl group is hydrolyzed, the resulting diester is water soluble;
ii) an esterase enzyme capable of catalyzing the hydrolysis of a water soluble glycerol diester to glycerol; and
iii) glycerol-detecting and -consuming reagents capable of producing a color change in the presence of glycerol; and the lipase is determined by detecting the rate of said color change at a time after said glycerol-detecting and -consuming reagents have consumed substantially all of said endogenous glycerol.

3. A reagent composition for the determination of lipase comprising a lipase substrate characterized in that the composition comprises:
(a) a lipase substrate which is a glyerol triester oil having in one of its two α-ester positions a long chain alkyl group having at least 8 carbon atoms and, in its two remaining ester positions, short chain alkyl groups such that, if the long chain alkyl group is hydrolyzed, the resulting diester is water soluble; and
(b) an esterase enzyme capable of catalyzing the hydrolysis of a water soluble glycerol diester to glycerol.

4. The reagent composition of claim 3 comprising glycerol-detecting and -consuming reagents capable of producing a color change in the presence of glycerol.

5. The method and composition according to claim 1, 2, 3 or 4 wherein said composition comprises a surfactant.

6. The method and composition according to claim 1, 2, 3 or 4 wherein said composition comprises a perfluorooctanoate ammonium salt surfactant.

7. A dry analytical element comprising a support and reagent zones for the determination of lipase, characterized in that the support has thereon:

(a) a zone comprising a lipase substrate which is a glyerol triester oil having in one of its two α-ester positions a long chain alkyl group having at least 8 carbon atoms and, in its two remaining ester positions, short chain alkyl groups such that, if the long chain alkyl group is hydrolyzed, the resulting diester is water soluble; and

(b) a zone comprising an esterase enzyme capable of catalyzing the hydrolysis of a water soluble glycerol diester to glycerol, said element comprising glycerol-detecting and -consuming reagents capable of producing a color change in the presence of glycerol.

8. A dry analytical element according to claim 7 characterized in that said glycerol-detecting and -consuming reagents are in a layer separate from said lipase substrate layer.

9. A dry analytical element according to claim 7 or 8 comprising a spreading layer.

10. A dry analytical element according to claim 9 characterised in that said spreading layer comprises a microcrystalline colloid and binder.

11. The dry analytical element of claim 7 comprising a spacer layer comprising a hydrophilic colliod and wherein the lipase substrate which is a glycerol triester oil having in one of its two α-ester positions a long chain alkyl group having at least 8 carbon atoms and, in its two remaining ester positions, short chain alkyl groups such that, if the long chain alkyl group is hydrolyzed, the resulting diester is water soluble.

12. The dry analytical element of claim 7 characterized in that said zones are discrete layers.

13. The method, composition and element according to claim 1, 2, 3, 4, 7, 11 or 12 characterized in that said substrate is 1-oleyl-2,3-diacetoyl glycerol.

14. The method composition and element according to claim 1, 2, 3, 4, 7, 11 or 12 characterized in that said esterase enzyme is a diacetinase from a *Bacillus subtilis* microorganism.

15. The method, composition and element according to claim 1, 2, 3, 4, 7, 11 or 12 characterised in that said esterase is a diacetinase from *Bacullus subtilis* ATCC No. 31954.

16. A dry analytical element according to claim 7, 11 or 12 characterized in that the layer containing said lipase substrate comprised a surfactant.

17. A dry analytical element according to claim 7, 11 or 12 characterized in that the layer containing said lipase substrate comprises a perfluorooctanoate ammonium salt surfactant.

**Patentansprüche**

1. Verfahren zur Bestimmung von Lipase in einer Probe mit den Verfahrensstufen:

(a) in-Kontakt-bringen der Probe mit einer Reagens-Zusammensetzung und

(b) Bestimmung der Lipasemenge, dadurch gekennzeichnet, daß die Reagens-Zusammensetzung aufweist:

i) ein Lipase-Substrat, das ein Glycerintriesteröl ist, das in einer seiner zwei α-Esterpositionen eine langkettige Alkylgruppe mit mindestens 8 Kohlenstoffatomen und in den zwei verbleibenden Esterpositionen kurzkettige Alkylgruppen aufweist, derart, daß, wenn die langkettige Alkylgruppe hydrolysiert wird, der erhaltene Diester wasserlöslich ist, und

ii) ein Esteraseenzym, das die Hydrolyse eines wasserlöslichen Glycerindiesters zu Glycerin zu katalysieren vermag, und daß die Lipase durch Ermittlung des Maßes, in dem Glycerin gebildet wird, bestimmt wird.

2. Verfahren nach Anspruch 1, dadurch gekennzeichnet, daß die Reagens-Zusammensetzung aufweist:

i) ein Lipase-Substrat, das ein Glycerintriesteröl ist, das in einer seiner zwei α-Esterpositionen eine langkettige Alkylgruppe mit mindestens 8 Kohlenstoffatomen und in den zwei verbleibenden Esterpositionen kurzkettige Alkylgruppen aufweist, derart, daß, wenn die langkettige Alkylgruppe hydrolysiert wird, der erhaltene Diester wasserlöslich ist;

ii) ein Esteraseenzym, das die Hydrolyse eines wasserlöslichen Glycerindiesters zu Glycerin zu katalysieren vermag und

iii) Glycerin ermittelnde und verbrauchende Reagenzien, die in Gegenwart von Glycerin einer Farbveränderung herbeizuführen vermögen, und daß die Lipase durch Ermittlung des Maßes der Farbveränderung zu einem Zeitpunkt, nach dem die Glycerin ermittelnden und verbaruchenden Reagenzien praktisch das gesamte endogene Glycerin verbraucht haben, bestimmt wird.

3. Reagens-Zusammensetzung für die Bestimmung von Lipase mit einem Lipase-Substrat, dadurch gekennzeichnet, daß die Zusammensetzung enthält:

a) ein Lipase-Substrat, das ein Glycerintriesteröl ist, das in einer seiner zwei α-Esterpositionen eine langkettige Alkylgruppe mit mindestens 8 Kohlenstoffatomen und in den zwei verbleibenden Esterpositionen kurzkettige Alkylgruppen aufweist, derart, daß, wenn die langkettige Alkylgruppe hydrolysiert wird, der erhaltene Diester wasserlöslich ist, und

b) ein Esteraseenzym, das die Hydrolyse eines wasserlöslichen Glycerindiesters zu Glycerin zu katalysieren vermag.

4. Reagens-Zusammensetzung nach Anspruch 3, dadurch gekennzeichnet, daß es Glycerin ermittelnde und verbrauchende Reagenzien enthält, die in Gegenwart von Glycerin eine Farbveränderung herbeizuführen vermögen.

5. Verfahren und Zusammensetzung nach Anspruch 1, 2, 3 oder 4, dadurch gekennzeichnet, daß die Zusammensetzung ein oberflächenaktives Mittel enthält.

6. Verfahren und Zusammensetzung nach Anspruch 1, 2, 3 oder 4, dadurch gekennzeichnet, daß die Zusammensetzung als oberflächenaktives Mittel ein Perfluoroctanoat-Ammoniumsalz enthält.

7. Analytisches Trockenelement mit einem Träger und Reagenszonen für die Bestimmung von Lipase, dadurch gekennzeichnet, daß auf dem Träger vorhanden sind:

(a) eine Zone mit einem Lipase-Substrat, das ein Glycerintriesteröl ist, das in einer seiner zwei α-Esterpositionen eine langkettige Alkylgruppe mit mindestens 8 Kohlenstoffatomen und in den zwei verbleibenden Esterpositionen kurzkettige Alkylgruppen aufweist, derart, daß, wenn die langkettige Alkylgruppe hydrolysiert wird, der erhaltene Diester wasserlöslich ist, und

(b) eine Zone mit einem Esteraseenzym, das die Hydrolyse eines wasserlöslichen Glycerindiesters zu Glycerin zu katalysieren vermag, und daß das Element Glycerin ermittelnde und verbrauchende Reagenzien enthält, die in Gegenwart von Glycerin eine Farbveränderung herbeizuführen vermögen.

8. Analytisches Trockenelement nach Anspruch 7, dadurch gekennzeichnet, daß die Glycerin ermittelnden und verbrauchenden Reagenzien in einer von der Lipase-Substratschicht getrennten Schicht enthalten sind.

9. Analytisches Trockenelement nach Anspruch 7 oder 8, dadurch gekennzeichnet, daß es eine Ausbreitschicht aufweist.

10. Analytisches Trockenelement nach Anspruch 9, dadurch gekennzeichnet, daß die Ausbreitschicht ein mikrokristallines Kolloid und ein Bindemittel enthält.

11. Analytisches Trockenelement nach Anspruch 7 mit einer Abstandsschicht mit einem hydrophilen Kolloid und worin das Lipase-Substrat ein Glycerintriesteröl ist, das in einer seiner zwei α-Esterpositionen eine langkettige Alkylgruppe mit mindestens 8 Kohlenstoffatomen und in den zwei verbleibenden Esterpositionen kurzkettige Alkylgruppen aufweist, derart, daß, wenn die langkettige Alkylgruppe hydrolysiert wird, der erhaltene Diester wasserlöslich ist.

12. Analytisches Trockenelement nach Anspruch 7, dadurch gekennzeichnet, daß die Zonen diskrete Schichten sind.

13. Verfahren, Zusammensetzung und Element nach Anspruch 1, 2, 3, 4, 7, 11 oder 12, dadurch gekennzeichnet, daß das Substrat aus 1-Oleyl-2,3-diacetoylglycerin besteht.

14. Verfahren, Zusammensetzung und Element nach Anspruch 1, 2, 3, 4, 7, 11 oder 12, dadurch gekennzeichnet, daß das Esteraseenzym eine Diacetinase von einem *Bacillus subtilis* Mikroorganismus ist.

15. Verfahren, Zusammensetzung und Element nach Anspruch 1, 2, 3, 4, 7, 11 oder 12, dadurch gekennzeichnet, daß das Esterase eine Diacetinase von *Bacillus subtilis* ATCC Nr. 31954 ist.

16. Analytisches Trockenelement nach Anspruch 7, 11 oder 12, dadurch gekennzeichnet, daß die Schicht, die das Lipase-Substrat enthält, auch ein oberflächenaktives Mittel aufweist.

17. Analytisches Trockenelement nach Anspruch 7, 11 oder 12, dadurch gekennzeichnet, daß die Schicht, die das Lipase-Substrat enthält, auch ein oberflächenaktives Mittel aus dem Perfluoroctanoat-Ammoniumsalz enthält.

**Revendications**

1. Procédé de détermination de lipase dans un échantillon, consistant à:

a) mettre en contact le dit échantillon avec une composition réactive, et,

b) déterminer le taux de lipase, caractérisé en ce que la composition réactive comprend:

i) un substrat pour la lipase, lequel substrat est une huile d'un triester de glycérol ayant sur une de ses deux positions α-ester un groupe alkyle à longue chaîne ayant au moins 8 atomes de carbone, et, sur ses deux positions esters restantes, des groupes alkyle à chaîne courte, de telle sorte que, si le groupe alkyle à longue chaîne est hydrolysé, le diester résultant est soluble dans l'eau; et

ii) une estérase capable de catalyser l'hydrolyse d'un diester de glycérol soluble dans l'eau en glycérol, la lipase étant déterminée par détection de la quantité de glycérol formé.

2. Procédé de la revendication 1, caractérisé en ce que la composition réactive comprend:

i) un substrat pour la lipase, lequel substrat est une huile d'un triester de glycérol ayant sur une de ses deux positions α-ester un groupe alkyle à longue chaîne ayant au moins 8 atomes de carbone et, sur ses positions esters restantes, des groupes alkyle à chaîne courte, de telle sorte que, si le groupe alkyle à longue chaîne est hydrolysé, le diester résultant est soluble dans l'eau;

ii) une estérase capable de catalyser l'hydrolyse d'un diester de glycérol soluble dans l'eau en glycérol; et

iii) des réactifs consommant et détectant le glycérol, capables de produire un changement de couleur en présence de glycérol; la lipase étant déterminée en détectant le taux de changement de couleur après que les dits réactifs consommant et détectant le glycérol ont consommé pratiquemment tout le glycérol endogène.

3. Composition réactive pour la détermination de lipase, comprenant un substrat pour la lipase, caractérisée en ce qu'elle comprend:

a) un substrat pour la lipase qui est une huile d'un triester de glycérol ayant sur une de ses 2 positions α-ester un groupe alkyle à longue chaîne ayant au moins 8 atomes de carbone et, sur ses positions esters restantes, des groupes alkyle à chaîne courte, de telle sorte que, si le groupe alkyle à longue chaîne est hydrolysé, le diester résultant est soluble dans l'eau, et

b) une estérase capable de catalyser l'hydrolyse d'un diester de glycérol soluble dans l'eau en glycérol.

4. Composition réactive de la revendication 3 comprenant des réactifs consommant et détectant le glycérol, capables de produire un changement de couleur en présence de glycérol.

5. Procédé et composition selon les revendications 1, 2, 3 ou 4, dans lesquels la dite composition comprend un agent tensio-actif.

6. Procédé et composition selon les revendications 1, 2, 3 ou 4, dans lesquels la dite composition comprend, comme agent tensio-actif, un perfluorooctanoate d'ammonium.

7. Produit d'analyse à sec, comprenant un support et des zones réactives pour la détermination de lipase, caractérisé en ce que se trouvent sur le support:

a) une zone comprenant un substrat pour la lipase, qui est une huile d'un triester de glycérol ayant sur l'une de ses deux positions α-ester un groupe alkyle à longue chaîne ayant au moins 8 atomes de carbone et, sur ses positions esters restantes, des groupes alkyle à chaîne courte, de telle sorte que, si le groupe alkyle à longue chaîne est hydrolysé, le diester résultant est soluble dans l'eau, et

b) une zone comprenant une estérase capable de catalyser l'hydrolyse d'un diester de glycérol soluble dans l'eau en glycérol, le dit produit comprenant des réactifs consommant et détectant le glycérol, capables de produire un changement de couleur en présence de glycérol.

8. Produit d'analyse à sec selon la revendication 7, caractérisé en ce que les réactifs consommant et détectant le glycérol sont dans une couche distincte de la couche de substrate pour la lipase.

9. Produit d'analyse à sec selon la revendication 7 ou 8, comprenant une couche d'étalement.

10. Produit d'analyse à sec selon la revendication 9, caractérisé en ce que la couche d'étalement comprend un colloïde microcristallin et un liant.

11. Produit d'analyse à sec de la revendication 7, comprenant une couche d'espacement formée d'un colloïde hydrophile, et dans lequel le substrat pour la lipase, qui est une huile d'un triester de glycérol ayant sur l'une de ses 2 positions α-ester un groupe alkyle à longue chaîne ayant au moins 8 atomes de carbone et, sur ses positions esters restantes, des groupes alkyle à chaîne courte, de telle sorte que, si le groupe alkyle à longue chaîne est hydrolysé, le diester résultant est soluble dans l'eau.

12. Produit d'analyse à sec de la revendication 7, caractérisé en ce que les zones sont des couches distinctes.

13. Procédé, composition et produit selon les revendications 1, 2, 3, 4, 7, 11 ou 12, caractérisés en ce que le dit substrat est le 1-oléyl-2,3-diacétoyl glycérol.

14. Procédé, composition et produit selon les revendications 1, 2, 3, 4, 7, 11 ou 12, caractérisés en ce que l'estérase est une diacétinase extraite du microorganisme *Bacillus subtilis*.

15. Procédé, composition et produit selon les revendications 1, 2, 3, 4, 7, 11 ou 12, caractérisés en ce que l'estérase est une diacétinase extraite de *Bacillus subtilis* ATCC n° 31954.

16. Produit d'analyse à sec selon les revendications 7, 11 ou 12, caractérisé en ce que le couche contenant le substrate pour la lipase comprend un agent tensio-actif.

17. Produit d'analyse à sec selon les revendications 7, 11 ou 12, caractérisé en ce que le couche contenant le substrat pour la lipase comprend, comme agent tensio-actif, le perfluorooctanoate d'ammonium.